# EUROPÄISCHE PATENTSCHRIFT

(11) **EP 3 030 542 B1**
(45) Veröffentlichungstag und Bekanntmachung des Hinweises auf die Patenterteilung: **21.06.2017**
(21) Anmeldenummer: 14744585.2
(22) Anmeldetag: 30.07.2014
(51) Int. Cl.: B01J 19/00, C07C 45/50, C07C 47/02, B01J 19/24, B08B 3/08

(54) **VERFAHREN ZUM ENTFERNEN VON COBALTABLAGERUNGEN IN EINEM OLEFIN HOCHDRUCKHYDROFORMYLIERUNG REAKTOR**
METHOD FOR REMOVING COBALT DEPOSITS IN A HIGH-PRESSURE OLEFIN HYDROFORMYLATION REACTOR
PROCÉDÉ D'ÉLIMINATION DES DÉPÔTS DE COBALT DANS UN RÉACTEUR D'HYDROFORMYLATION D'OLÉFINES À HAUTE PRESSION

(30) Priorität: 08.08.2013 EP 13179688
(43) Veröffentlichungstag der Anmeldung: 15.06.2016
(73) Patentinhaber: BASF SE, 67056 Ludwigshafen am Rhein (DE)
(72) Erfinder: RUDOLPH, Jens, 67056 Ludwigshafen (DE); PAPP, Rainer, 67346 Speyer (DE)
(74) Vertreter: BASF IP Association
(86) Internationale Anmeldenummer: PCT/EP2014/066372
(87) Internationale Veröffentlichungsnummer: WO 2015/018710

(56) Entgegenhaltungen:
- EP-A1- 0 024 761
- DE-C- 750 498
- FR-A- 1 089 983
- US-B2- 7 910 782

## Beschreibung

Die cobaltkatalysierte Hochdruckhydroformylierung ist ein wichtiger Verfahrensschritt bei der Herstellung höher verzweigter Alkohole aus höher verzweigten Olefinen, wie beispielsweise der Herstellung von Isononanol aus Isoocten.

Bei der cobaltkatalysierten Hochdruckhydroformylierung werden Kohlenstoffmonoxid und Wasserstoff in Gegenwart des Katalysatormetalls Cobalt, das homogen als HCo(CO)₄ wirksam ist, an die C=C-Doppelbindung des Olefins addiert, wobei ein Aldehyd gebildet wird. Der Aldehyd weist im Vergleich zum Olefin ein zusätzliches Kohlenstoffatom auf. Die Umsetzung erfolgt im Allgemeinen in einem Reaktor bei Temperaturen von 120 bis 240 °C unter einem Synthesegasdruck von 150 bis 400 bar.

Zur cobaltkatalysierten Hochdruckhydroformylierung wurden mehrstufige Prozesse entwickelt, die es ermöglichen, den mit den Hydroformylierungsprodukten aus dem Reaktor austretenden Katalysator von den Hydroformylierungsprodukten abzutrennen und in die Hydroformylierungsreaktion zurückzuführen. Ein solcher Prozess kann die 4 Verfahrensstufen der Vorcarbonylierung, der Katalysatorextraktion, der Olefinhydroformylierung und der Entcobaltung umfassen.

Bei der Vorcarbonylierung wird ausgehend von einer wässrigen Cobaltsalzlösung, die z. B. Cobaltformiat oder Cobaltacetat enthält, durch Umsetzung mit Kohlenstoffmonoxid und Wasserstoff der für die Hydroformylierung benötigte Katalysator HCo(CO)₄ hergestellt. Bei der Katalysatorextraktion wird der in der ersten Verfahrensstufe hergestellte Katalysator aus der wässrigen Phase mit einer organischen Phase, vorzugsweise mit dem zu hydroformylierenden Olefin, extrahiert. Nach der Phasentrennung wird die mit dem Katalysator beladene organische Phase der Olefinhydroformylierung zugeführt. Bei der Entcobaltung wird die organische Phase des Reaktoraustritts von den Cobaltcarbonylkomplexen in Gegenwart von Prozesswasser, das Ameisensäure oder Essigsäure enthalten kann, durch Behandlung mit Sauerstoff oder Luft befreit. Dabei wird der Katalysator oxidativ zerstört und die erhaltenen Cobaltsalze in die wässrige Phase zurückextrahiert. Die aus der Entcobaltung erhaltene wässrige Cobaltsalzlösung wird in die erste Verfahrensstufe, die Vorcarbonylierung, zurückgeführt. Die Vorcarbonylierung, die Katalysatorextraktion und die Olefinhydroformylierung kann auch in einem einstufigen Prozess im Hydroformylierungsreaktor durchgeführt werden.

Es ist bekannt, dass HCo(CO)₄ bei der im Hydroformylierungsreaktor vorherrschenden hohen Temperatur nur bei hohem Partialdruck des CO stabil ist (siehe New Synthesis with Carbon Monoxide, J. Falbe, Springer Verlag 1980, Seite 17, Fig. 1.9). Im Reaktor bilden sich in der Regel unterdurchschnittlich stark durchmischte Zonen aus. Insbesondere in weniger stark durchmischten Zonen kann die Temperatur erhöht und/oder der Partialdruck des CO verringert sein, so dass sich darin HCo(CO)₄ zersetzt. Infolge der Zersetzung fällt metallisches Cobalt aus, wodurch Cobaltablagerungen entstehen. Die Cobaltablagerungen können dazu führen, dass der Reaktor schlechter durchmischt wird. Die schlechtere Durchmischung begünstigt wiederum die weitere Bildung von Cobaltablagerungen. Letztlich führt die abnehmende Durchmischung des Reaktors zu einer Verringerung der Ausbeute an Hydroformylierungsprodukt.
Die mechanische Entfernung von Cobaltablagerungen ist z. B. unter Einsatz eines Hochdruckwasserstrahls bei geöffnetem Reaktordeckel möglich. Die Öffnung des Reaktordeckels und das anschließende druckdichte Wiederverschließen ist jedoch mit hohem technischen Aufwand verbunden. US 7910782 B1 und FR 1089983 A beschreiben ein Verfahren zur Wiedergewinnung von Cobalt aus dem Produkt einer Cobalt-katalysierten Hydroformylierungsreaktion, wobei Salpetersäure eingesetzt wird. Aus der EP 0 024 761 A1 ist bekannt, dass Übergangsmetallablagerungen, die sich an den Innenwänden eines Hydroformylierungsreaktors bilden, durch Reinigung mit korrosiven Flüssigkeiten, wie z. B. wässriger Salpetersäure, entfernt werden können.
Wässrige Salpetersäure lässt sich ohne Öffnung des Reaktordeckels in den Reaktor einbringen. Sie kann durch Öffnungen, wie z. B. Rohrstutzen, in den Reaktor eingeleitet werden. Beim Lösen von Cobaltablagerungen durch wässrige Salpetersäure bildet sich ein stickoxidhaltiges Abgas.
Der vorliegenden Erfindung liegt die Aufgabe zugrunde, ein Verfahren zur Entfernung von Cobaltablagerungen aus dem Hydroformylierungsreaktor durch wässrige Salpetersäure bereitzustellen, bei dem die Freisetzung des stickoxidhaltigen Abgases kontrolliert verläuft und das die Säurekapazität der eingesetzten Salpetersäure weitgehend ausnutzt.
Diese Aufgabe wird gelöst durch ein Verfahren zum Entfernen von Cobaltablagerungen in einem Reaktor zur cobaltkatalysierten Hochdruckhydroformylierung von Olefinen durch Behandlung mit wässriger Salpetersäure, wobei man den Reaktor zumindest teilweise mit wässriger Salpetersäure befüllt, die Säurekonzentration und/oder Cobaltionenkonzentration der wässrigen Salpetersäure im Verlauf der Behandlung misst und, wenn die zeitliche Veränderung der Säurekonzentration und/oder der Cobaltionenkonzentration unter einen vorgegebenen Grenzwert sinkt, die Temperatur der wässrigen Salpetersäure während der Behandlung erhöht.

Beim Einwirken der wässrigen Salpetersäure werden die Cobaltablagerungen gelöst, wobei das elementare Cobalt zu Cobalt(II)-Verbindungen oxidiert wird, die in wässriger Salpetersäure gut löslich sind. Salpetersäure wird unter Freisetzung eines stickoxidhaltigen Abgases reduziert. Demzufolge nimmt dabei die Salpetersäurekonzentration während der Behandlung kontinuierlich ab und die Cobaltionenkonzentration kontinuierlich zu. Erfindungsgemäß erhöht man die Temperatur während der Behandlung, um bei verringerter Salpetersäurekonzentration eine ausreichende Reaktionsgeschwindigkeit aufrecht zu erhalten.

Demzufolge lassen sich durch kontrollierte Erhöhung der Temperatur eine konstante Reaktionsgeschwindigkeit und damit auch ein gleichmäßiger Strom des gebildeten stickoxidhaltigen Abgases einstellen. Ein gleichmäßiger Strom stickoxidhaltigen Abgases vereinfacht es, das stickoxidhaltige Abgas so zu behandeln, dass es nicht in die Atmosphäre gelangen kann. In einer erfindungsgemäß bevorzugten Ausführungsform erhöht man die Temperatur der wässrigen Salpetersäure im Verlauf der Behandlung, wenn die zeitliche Veränderung der Säurekonzentration und/oder der Cobaltionenkonzentration eine Abnahme der Reaktionsgeschwindigkeit anzeigt. Es gelingt so, einen gleichmäßigen Strom stickoxidhaltigen Abgases einzustellen.

In einer bevorzugten Ausführungsform des Verfahrens erhöht man die Temperatur der wässrigen Salpetersäure von einer ersten Temperatur im Bereich von 10 bis 40 °C auf eine Endtemperatur im Bereich von 60 bis 80 °C, bevorzugt 70 bis 80 °C, besonders bevorzugt 75 bis 80 °C. Um ein Sieden der wässrigen Salpetersäure zu verhindern, erhöht man die Temperatur der wässrigen Salpetersäure während der Behandlung vorzugsweise nicht über 80°C. Die wässrige Salpetersäure wird bei einer ersten Temperatur, vorzugsweise Umgebungstemperatur, in den Reaktor eingeleitet. Zunächst erfolgt die Behandlung vorzugsweise ohne aktive Zufuhr von Wärme. Man erhöht die Temperatur anschließend schrittweise oder kontinuierlich auf eine Endtemperatur im Bereich von 60 bis 80 °C. Bevorzugt erhöht man die Temperatur schrittweise um 6 bis 40 °C, bevorzugt 8 bis 30 °C, besonders bevorzugt 10 bis 25 °C, bis die Endtemperatur erreicht ist. Die Temperaturerhöhung erfolgt durch aktive Zufuhr von Wärme.

Geeignete, druckfeste Reaktionsapparaturen für die Hochdruckhydroformylierung sind dem Fachmann bekannt. Dazu zählen die allgemein üblichen Reaktoren für Gasflüssig-Reaktionen, wie z. B. Rohrreaktoren, Rührkessel, Gasumlaufreaktoren, Blasensäulen usw., die gegebenenfalls durch Einbauten nochmals unterteilt sein können. Geeignet ist beispielsweise ein senkrecht stehender Hochdruck-Blasensäulenreaktor, der gegebenenfalls mit koaxialen rohrförmigen Einbauten versehen ist. Als Reaktor wird typischerweise ein Hochdruckrohr (siehe z. B. DE 19 38 102) mit angeflanschten Deckeln verwendet, das zumindest zwei Öffnungen aufweist. Die Öffnungen sind vorzugsweise in Form von Rohrstutzen ausgeprägt und dienen zur Einleitung der Edukte und des Katalysators sowie zur Ausleitung der Produkte. Die Reaktorwand besteht aus einem druckfesten Stahl und ist mit einer Auskleidung aus Edelstahl (V2A oder V4A) versehen.

Durch das erfindungsgemäße Verfahren braucht der Reaktordeckel nicht geöffnet zu werden, da die wässrige Salpetersäure bei geschlossenem Reaktordeckel durch andere Öffnungen in den Reaktor eingeleitet werden kann. Bei den Öffnungen handelt es sich vorzugsweise um Öffnungen zur Einleitung der Edukte und des Katalysators sowie zur Ausleitung der Produkte. Vorzugsweise handelt es sich bei diesen Öffnungen um leicht zu öffnende Rohrstutzen. Dies bietet gegenüber der mechanischen Entfernung von Cobaltablagerungen, wie z. B. durch einen Hochdruckwasserstrahl, den Vorteil, dass der mit der Öffnung des Reaktordeckels und mit dem anschließenden druckdichten Wiederverschließen verbundene hohe technische Aufwand entfällt.

Es ist bevorzugt, das stickoxidhaltige Abgas so zu behandeln, dass es nicht in die Atmosphäre gelangen kann. In einer Ausführungsform vereinigt man ein stickoxidhaltiges Abgas, das beim Lösen der Cobaltablagerungen in der wässrigen Salpetersäure entsteht, mit einem molekularen Sauerstoff enthaltenden Gas und absorbiert das Abgas zumindest teilweise in einer wässrigen Flüssigkeit. Die wässrige Flüssigkeit ist z. B. Wasser selbst oder eine wässrige alkalische Lösung, bevorzugt eine wässrige Natriumhydroxid-Lösung. Die Absorption des Abgases erfolgt vorzugsweise bei einer Temperatur von 10 bis 50 °C.

Geeigneterweise führt man dem Abgas in einer Waschkolonne die wässrige Flüssigkeit entgegen und leitet in das Abgas und/oder in die Waschkolonne ein molekularen Sauerstoff enthaltendes Gas ein. Bei der Waschkolonne handelt es sich bevorzugt um eine gepackte Kolonne. Vorzugsweise enthält die Kolonne eine Füllkörperschüttung, wobei man unterhalb der Schüttung das Abgas einleitet und oberhalb der Schüttung die wässrige Flüssigkeit aufgibt. Bevorzugt umfasst die Schüttung aus Edelstahl bestehende Pall-Ringe. Die Füllkörperschüttung intensiviert den Kontakt zwischen dem Abgas, der die wässrige Flüssigkeit und gegebenenfalls dem molekularen Sauerstoff enthaltenden Gas.

Das stickoxidhaltige Abgas enthält einen hohen Anteil NO, das schlechter wasserlöslich ist als NO₂. Durch Vereinigen des stickoxidhaltigen Abgases mit dem molekularen Sauerstoff enthaltenden Gas wird zumindest ein Teil des NO zum besser wasserlöslichen NO₂ oxidiert. Der sich einstellende Oxidationsgrad beträgt vorzugsweise wenigstens 50 %, z. B. 50 bis 60 %. Der Oxidationsgrad ist definiert als Volumenanteil von NO₂ im Verhältnis zum Gesamtvolumen NO + NO₂. Als molekularen Sauerstoff enthaltendes Gas verwendet man vorzugsweise Luft.

Das molekularen Sauerstoff enthaltende Gas wird vorzugsweise ebenfalls unterhalb der Schüttung eingeleitet oder vor der Kolonne dem stickoxidhaltigen Abgas zugemischt. Das Zumischen erfolgt vorzugsweise unter solchen Bedingungen, dass eine ausreichende Verweilzeit zur Einstellung des angestrebten Oxidationsgrades resultiert (Daten zur Reaktionskinetik: M. Bodenstein: Die Geschwindigkeit der Reaktion zwischen Stickoxyd und Sauerstoff, Z. f. Elektroch. 24, S. 184, 1918). Dies kann beispielsweise durch Zumischen des molekularen Sauerstoff enthaltenden Gases in ein Rohr oder einen Behälter, der vom stickoxidhaltigen Abgas mit niedriger Strömungsgeschwindigkeit durchströmt wird, erreicht werden.

Das bei der Behandlung des Abgases mit Wasser anfallende Abwasser führt man bevorzugt einer Abwasserbehandlung zu.

Der Reaktor wird zumindest teilweise mit wässriger Salpetersäure befüllt. Bevorzugt wälzt man die wässrige Salpetersäure während der Behandlung im Reaktor um. Durch das Umwälzen wird eine Durchmischung der Salpetersäure erreicht und durch Vermeidung von Konzentrationsgradienten die Auflösungsgeschwindigkeit der Ablagerungen erhöht. Die im Reaktor befindliche wässrige Salpetersäure wird so schnell umgewälzt, dass diese im Mittel mindestens alle drei Stunden, vorzugsweise alle zwei Stunden, bevorzugt jede Stunde einmal ausgetauscht wird (also bei 10 m³ wässriger Salpetersäure wird bevorzugt mit 10 m³/h umgewälzt). Vorzugsweise erwärmt man die wässrige Salpetersäure beim Umwälzen, wobei man einen außerhalb des Reaktors geführten Strom der wässrigen Salpetersäure erwärmt. Die Wärme führt man bevorzugt über einen Wärmetauscher oder eine elektrische Heizung zu.

Alternativ kann man ein innenliegendes Kühlsystem des Reaktors zum Erwärmen der wässrigen Salpetersäure nutzen. Das innenliegende Kühlsystem dient während des Produktivbetriebs zum Abführen der Reaktionswärme. Es ist in der Regel so ausgelegt, dass der Reaktorinhalt mit dem innenliegenden Kühlsystem auch aufgeheizt werden kann.

In einer geeigneten Ausführungsform wird ein Strom der Salpetersäure am unteren Ende des Reaktors abgezogen und am oberen Ende des Reaktors wieder aufgegeben. Man setzt bevorzugt eine Pumpe ein, um die abgezogene Salpetersäure aufzugeben. Das Wiederaufgeben erfolgt vorzugsweise so, dass Cobaltablagerungen, die sich oberhalb des Flüssigkeitsspiegels der Salpetersäure im Reaktor befinden, möglichst vollständig mit der wässrigen Salpetersäure benetzt werden. Vorzugsweise führt man die wässrige Salpetersäure vor Eintritt in die Pumpe durch einen Filter, der den Eintritt von Feststoffen, wie z. B. losgelösten partikulären Ablagerungen, in die Pumpe verhindert. In einer bevorzugten Ausführungsform wird der Reaktor zu maximal 70 %, bevorzugt maximal 60 %, besonders bevorzugt maximal 50 % mit der wässrigen Salpetersäure befüllt. Vorzugsweise wird der Reaktor wenigstens zu 1 %, bevorzugt wenigstens zu 5 %, besonders bevorzugt wenigstens zu 10 % mit der wässrigen Salpetersäure befüllt.

In einer alternativen Ausführungsform wird der Reaktor komplett mit wässriger Salpetersäure befüllt, wobei man einen Überlaufbehälter zum Auffangen überlaufender Salpetersäure nutzt. Bevorzugt setzt man einen Nachreaktor oder einen Druckabscheider als Überlaufbehälter ein. Aus dem Überlaufbehälter leitet man die Säure aus und führt sie in den Reaktor zurück. In dieser Ausführungsform führt man die Salpetersäure bevorzugt zurück, indem man sie am unteren Ende des Reaktors einleitet. Man setzt bevorzugt eine Pumpe ein, um die Salpetersäure zurückzuführen. Vorzugsweise führt man die wässrige Salpetersäure vor Eintritt in die Pumpe durch einen Filter, der den Eintritt von Feststoffen in die Pumpe verhindert.

Die Konzentration der initial eingesetzten wässrigen Salpetersäure liegt vorzugsweise im Bereich von 100 bis 200 g HNO₃ l⁻¹.
Beim erfindungsgemäßen Verfahren wird eine ganz oder teilweise erschöpfte wässrige Salpetersäure gebildet. Der Säuregehalt der erschöpften wässrigen Salpetersäure reicht nicht aus, um weitere Cobaltablagerungen mit nennenswerter Reaktionsgeschwindigkeit aufzulösen. Die wässrige Salpetersäure ist in der Regel erschöpft, wenn die Konzentration der Salpetersäure unter 25 g HNO₃ l⁻¹ sinkt. Der Säuregehalt einer teilweise erschöpften wässrigen Salpetersäure reicht aus, um weitere Cobaltablagerungen mit nennenswerter Reaktionsgeschwindigkeit aufzulösen. Die wässrige Salpetersäure ist in der Regel teilweise erschöpft, wenn die Konzentration der Salpetersäure unterhalb der initial eingesetzten Salpetersäure und oberhalb 25 g HNO₃ l⁻¹ liegt. Eine teilweise erschöpfte wässrige Salpetersäure kann aufbewahrt werden, um zu einem späteren Zeitpunkt erneut als wässrige Salpetersäure in einem erfindungsgemäßen Verfahren eingesetzt zu werden. Bei dem erfindungsgemäßen Verfahren misst man die Säurekonzentration und/oder Cobaltionenkonzentration der wässrigen Salpetersäure im Verlauf der Behandlung. Die Säurekonzentration misst man bevorzugt durch Säure-Base-Titration oder mittels einer pH-Sonde. Die Cobaltionenkonzentration misst man bevorzugt durch komplexometrische Titration. Die zeitliche Veränderung der Säurekonzentration und/oder der Cobaltionenkonzentration ergibt sich aus den Messungen, die man zu unterschiedlichen Zeitpunkten im Verlauf der Behandlung durchführt. Vorzugsweise führt man die Messungen im Abstand von 10-300 Minuten, bevorzugt 20-180 Minuten, besonders bevorzugt 30-120 Minuten durch. Somit erhöht man bei dem erfindungsgemäßen Verfahren die Temperatur der wässrigen Salpetersäure, wenn die zeitliche Veränderung der Säurekonzentration und/oder der Cobaltionenkonzentration unter einen vorgegebenen Grenzwert sinkt. Man kann die Temperatur der wässrigen Salpetersäure erhöhen, sobald die zeitliche Veränderung der Säurekonzentration unter einen Grenzwert von 1 g HNO₃ l⁻¹h⁻¹ fällt. Man kann die Temperatur der wässrigen Salpetersäure erhöhen, wenn die zeitliche Veränderung der Cobaltionenkonzentration unter einen Grenzwert von 0,5 g l⁻¹h⁻¹ fällt. Bevorzugt erhöht man die Temperatur schrittweise um 6 bis 40 °C, bevorzugt 8 bis 30 °C, besonders bevorzugt 10 bis 25 °C, bis die Endtemperatur erreicht ist. Die Temperaturerhöhung erfolgt durch aktive Zufuhr von Wärme. In einer bevorzugten Ausführungsform des Verfahrens erhöht man die Temperatur der wässrigen Salpetersäure von einer ersten Temperatur im Bereich von 10 bis 40 °C auf eine Endtemperatur im Bereich von 60 bis 80 °C, bevorzugt 70 bis 80 °C, besonders bevorzugt 75 bis 80 °C.
Sobald die Säurekonzentration unter einen vorgegebenen Grenzwert sinkt und/oder die Cobaltionenkonzentration über einen vorgegebenen Grenzwert steigt, ist die Salpetersäure erschöpft. Die erschöpfte wässrige Salpetersäure wird aus dem Reaktor abgelassen. Man lässt die erschöpfte wässrige Salpetersäure dann bevorzugt aus dem Reaktor ab, sobald die Salpetersäurekonzentration unter 25 g HNO₃ l⁻¹ sinkt und/oder die Cobaltionenkonzentration über 85 g l⁻¹ steigt. Oberhalb einer Cobaltionenkonzentration von 85 g l⁻¹ kann die Löslichkeitsgrenze von Co(NO₃)₂ überschritten werden.
Sind die Cobaltablagerungen noch nicht vollständig entfernt, wenn erschöpfte wässrige Salpetersäure aus dem Reaktor abgelassen wird, wird der Reaktor vorzugsweise mit frischer wässriger Salpetersäure befüllt und das Verfahren wiederholt.
Wenn bei der höchsten Temperatur die Säurekonzentration der wässrigen Salpetersäure oberhalb des vorgegebenen Grenzwertes ist, aber die zeitliche Veränderung der Säurekonzentration und/oder der Cobaltionenkonzentration unter vorgegebene Grenzwerte sinken, ist davon auszugehen, dass die Cobaltablagerungen im wesentlichen entfernt sind.

Bevorzugt setzt man die wässrige Salpetersäure in stöchiometrischem Überschuss, bezogen auf die zu lösenden Cobaltablagerungen, ein. Vorzugsweise setzt man zumindest 3 Mol, insbesondere zumindest 5 Mol, besonders bevorzugt zumindest 8 Mol, ganz besonders bevorzugt zumindest 10 Mol verfügbare Salpetersäure, gerechnet als HNO₃, pro Mol Cobalt ein. Die Menge des abgelagerten Cobalts lässt sich anhand von Verfahrensparametern, wie der Dauer und der Temperatur des vorangegangenen Produktivbetriebs oder aus der im Verlauf des vorangegangenen Produktivbetriebs ergänzten Cobaltmenge abschätzen. Als "verfügbare Salpetersäure" soll der fiktive Anteil der wässrigen Salpetersäure angesehen werden, der bei einer Konzentration oberhalb der oben definierten Erschöpfungskonzentrationsgrenze von 25 g/l verfügbar ist. Demgemäß enthielte eine wässrige Salpetersäure mit 100 g HNO₃/l eine verfügbare Salpetersäure von (100-25)= 75 g/l. Anhand des Volumens der eingesetzten wässrigen Salpetersäure (und des Molekulargewichts von HNO₃) kann die Stoffmenge (in Mol) der verfügbaren Salpetersäure bestimmt werden. Wird die erfindungsgemäße Behandlung in mehreren Schritten durchgeführt, z. B. indem zunächst eine teilweise erschöpfte wässrige Salpetersäure eingesetzt wird, die - sobald sie erschöpft ist - abgelassen und durch frische wässrige Salpetersäure ersetzt wird, gehen in die Berechnung der verfügbaren Salpetersäuremenge die Beiträge der einzelnen Schritte ein.

Im Allgemeinen unterbricht man die cobaltkatalysierte Hydroformylierungsreaktion zur Vorbereitung des Reaktors für die erfindungsgemäße Behandlung mit wässriger Salpetersäure. Vorzugsweise entspannt man hierbei den Reaktor und lässt das im Reaktor befindliche Reaktionsgemisch ab. Dabei verbleiben nicht abfließende Rückstände des Reaktionsgemisches im Reaktor, die organische Verbindungen, wie z. B. Olefin und/oder Aldehyde umfassen. Bevorzugt entfernt man die Rückstände vor der erfindungsgemäßen Behandlung mit wässriger Salpetersäure, indem man den Reaktor mit Wasser oder Wasserdampf spült.

In einer Ausführungsform des Verfahrens reinigt man den Reaktor nach der Behandlung mit wässriger Salpetersäure mit einem Wasserstrahl, um die Entfernung der Cobaltablagerungen zu vervollständigen. Wenn das abgelagerte Cobalt mit Kohlenstoff vermischt ist oder wenn das Cobalt sich in größeren Agglomeraten abgelagert hat, kann nach der Behandlung mit wässriger Salpetersäure eine Behandlung mit einem Wasserstrahl, bevorzugt einem Hochdruckwasserstrahl, erforderlich sein, um die Entfernung der Ablagerungen zu vervollständigen. Um die nach der Behandlung mit der wässrigen Säure im Reaktor verbleibenden Ablagerungen zu entfernen, reicht es aus, den Wasserstrahl durch leicht zu öffnende Öffnungen, wie z. B. Rohrstutzen, in den Reaktor zu führen. Folglich führt man das Verfahren auch in dieser Ausführungsform durch, ohne den Reaktor zu öffnen, wodurch der mit der Öffnung des Reaktordeckels und mit dem anschließenden druckdichten Wiederverschließen verbundene hohe technische Aufwand entfällt.

Bei einem Hochdruckwasserstrahl handelt es sich um einen Wasserstrahl, den man durch Anlegen eines Drucks von 2 bis 500 bar (absolut) erzeugt.

Die Rückstände, die nach dem Ablassen der wässrigen Salpetersäure im Reaktor verbleiben, lassen sich durch Wasser oder Wasserdampf nahezu vollständig entfernen. In einer bevorzugten Ausführungsform des Verfahrens spült man den Reaktor mit Wasser oder Wasserdampf, nachdem die Behandlung mit wässriger Salpetersäure durchgeführt wurde. Im Reaktor verbleibende Wasserrückstände beeinträchtigen den nachfolgenden Hydroformylierungsprozess nicht. Demzufolge ist zwischen dem Reinigungsbetrieb (Entfernen von Cobaltablagerungen) und dem Produktivbetrieb (Hydroformylierungsprozess) nur ein geringer Aufwand erforderlich.

Durch die erfindungsgemäße Temperaturführung kommt die Oxidation der Cobaltablagerungen auch dann noch nicht zum Erliegen, wenn die Säurekonzentration der wässrigen Salpetersäure schon verringert und die Cobaltionenkonzentration in der wässrigen Salpetersäure angestiegen ist. Folglich fällt, gegebenenfalls nach mehrmaliger Verwendung einer teilweise erschöpften wässrigen Salpetersäure, im erfindungsgemäßen Verfahren eine erschöpfte wässrige Salpetersäure mit hoher Cobaltionenkonzentration von bis zu 85 gl⁻¹ an. Aus dieser lässt sich Cobalt mit geringem Aufwand zurückgewinnen. Demzufolge ist eine Ausführungsform des Verfahrens in der man Cobalt aus der erschöpften wässrigen Salpetersäure zurückgewinnt, besonders bevorzugt.

Bevorzugt fällt man aus der anfallenden erschöpften wässrigen Salpetersäure ein Cobaltsalz, das Hydroxid- und/oder Oxidanionen enthält, aus, trennt das Cobaltsalz ab und setzt das Cobaltsalz mit Essigsäure zu einer Cobaltacetat-Lösung um. Besonders bevorzugt filtriert man die erschöpfte wässrige Salpetersäure zunächst, um enthaltenen Feststoff abzutrennen, und stellt das Filtrat mit Natronlauge neutral oder leicht basisch ein, wobei Cobalt als Cobalthydroxidhydrat ausfällt. Man filtriert das ausgefällte Cobalthydroxidhydrat ab und trocknet es über eine Filterpresse. Den Filterkuchen setzt man durch Versetzen mit Essigsäure wieder in eine Cobaltacetat-Lösung um. Es ist vorteilhaft, wenn die Cobaltionenkonzentration in der anfallenden erschöpften wässrigen Salpetersäure möglichst hoch ist.

Vorzugsweise setzt man das zurückgewonnene Cobaltacetat wieder in einem Hydroformylierungsverfahren ein.

### Beispiel 1

Ein Hydroformylierungsreaktor mit einem inneren Durchmesser von 1,5 m und einer inneren Höhe von 18 m wurde gründlich geleert und anschließend mehrmals mit heißem Wasser gefüllt und wieder entleert, um Reste von organischem Material weitgehend zu entfernen. Der Reaktor wurde mit 10 m³ wässriger Salpetersäure (65 g HNO₃/l) gefüllt, die 64 g/l Cobalt enthielt, und eine Temperatur von 20 °C aufwies. Anschließend wurde die Säure am Boden des Reaktors mit Hilfe einer Pumpe abgezogen und oben wieder in den Reaktor gegeben. Auf diese Weise wurden ca. 8 m³/h umgepumpt.

Die entstehenden Abgase wurden, zusammen mit 20 Nm³/h Luft, von unten in eine Kolonne geleitet, die einen Innendurchmesser von 350 mm, eine innere Höhe von 5000 mm und eine Schüttung mit 25 mm Pallringen mit einer Höhe von 2500 mm aufwies. Die Kolonne wurde von oben mit 5 m³/h Wasser beaufschlagt. Das anfallende Abwasser wurde der Abwasserreinigungsanlage zugeführt.

Die wässrige Salpetersäure wurde im Reaktor zunächst auf eine Temperatur von 35 °C erwärmt. Innerhalb von 10 Stunden fiel die Konzentration der wässrigen Salpetersäure auf 26 g/l und die Cobaltionenkonzentration stieg auf 72 g/l. Nachdem sich die Cobaltionenkonzentration zwei Stunden lang nicht veränderte wurde die Lösung abgelassen.

Der Reaktor wurde so lange mit entionisiertem Wasser gespült bis das ablaufende Wasser neutral war.

### Beispiel 2

Ein Hydroformylierungsreaktor mit einem inneren Durchmesser von 1 m und einer inneren Höhe von 18 m wurde gründlich geleert und anschließend mehrmals mit heißem Wasser gefüllt und wieder entleert, um Reste von organischem Material weitgehend zu entfernen. Der Reaktor wurde mit 20 m³ wässriger Salpetersäure (161 g HNOs/l) gefüllt, die 29 g/l Cobalt enthielt und eine Temperatur von 20 °C aufwies, wobei ein Teil der Säure in den Nachreaktor überlief. Anschließend wurde die Säure am Boden des Nachreaktors mit Hilfe einer Pumpe abgezogen und in den unteren Teil des Reaktors zurückgeführt. Auf diese Weise wurden ca. 8 m³/h umgepumpt.

Die entstehenden Abgase wurden, zusammen mit 20 Nm³/h Luft, von unten in eine Kolonne geleitet, die einen Innendurchmesser von 350 mm, eine innere Höhe von 5000 mm und eine Schüttung mit 25 mm Pallringen mit einer Höhe von 2500 mm aufwies. Die Kolonne wurde von oben mit 5 m³/h Wasser beaufschlagt. Das anfallende Abwasser wurde der Abwasserreinigungsanlage zugeführt.

Die wässrige Salpetersäure wurde im Reaktor zunächst auf eine Temperatur von 50 °C erwärmt. Innerhalb von 24 Stunden nahm die Säurekonzentration auf 120 g/l ab und die Cobaltionenkonzentration stieg auf 40 g/l. Nach weiteren 18 Stunden sank die Säurekonzentration auf 107 g/l und änderte sich dann innerhalb von drei Stunden nicht mehr. Nach Erhöhung der Temperatur auf 70 °C sank die Säurekonzentration innerhalb von 12 Stunden auf 93 g/l und die Cobaltionenkonzentration stieg auf 45 g/l. Nachdem sich die Konzentrationen bei zwei Bestimmungen innerhalb von sechs Stunden nicht mehr änderten, wurde die Lösung abgelassen.

Der Reaktor wurde so lange mit Kondensat gespült bis das ablaufende Kondensat neutral war.

### Beispiel 3

Ein Hydroformylierungsreaktor mit einem inneren Durchmesser von 1,5 m und einer inneren Höhe von 18 m wurde gereinigt, indem 10 m³ 20%ige Salpetersäure eingesetzt wurden. Um den Reinigungserfolg zu kontrollieren, wurde die interne Umlaufzeit im Reaktor durch eine radioaktive Stoßmarkierung gemessen. Als Indikator wurde das Natriumisotop ²⁴Na verwendet.

Die Messungen wurden in zwei Betriebszuständen durchgeführt, einmal vor dem Entfernen der Cobaltablagerungen und einmal nach dem Reinigen. Die Geschwindigkeit des im Einsteckrohr aufwärts strömenden Indikators wurde vor dem Reinigen zu 25 cm/s bestimmt, nach dem Reinigen wurden 41 cm/s erreicht.

## Patentansprüche

1. Verfahren zum Entfernen von Cobaltablagerungen in einem Reaktor zur cobaltkatalysierten Hochdruckhydroformylierung von Olefinen durch Behandlung mit wässriger Salpetersäure, wobei man den Reaktor zumindest teilweise mit wässriger Salpetersäure befüllt, die Säurekonzentration und/oder Cobaltionenkonzentration der wässrigen Salpetersäure im Verlauf der Behandlung misst und, wenn die zeitliche Veränderung der Säurekonzentration und/oder der Cobaltionenkonzentration unter einen vorgegebenen Grenzwert sinkt, die Temperatur der wässrigen Salpetersäure während der Behandlung erhöht.

2. Verfahren nach Anspruch 1, wobei man ein stickoxidhaltiges Abgas, das beim Lösen der Cobaltablagerungen in der wässrigen Salpetersäure entsteht, mit einem molekularen Sauerstoff enthaltenden Gas vereinigt und das Abgas zumindest teilweise in einer wässrigen Flüssigkeit absorbiert.

3. Verfahren nach Anspruch 2, wobei man dem Abgas in einer Waschkolonne die wässrige Flüssigkeit entgegen führt und in das Abgas und/oder in die Waschkolonne ein molekularen Sauerstoff enthaltendes Gas einleitet.

4. Verfahren nach einem der vorhergehenden Ansprüche, wobei man die wässrige Salpetersäure während der Behandlung im Reaktor umwälzt.

5. Verfahren nach einem der vorhergehenden Ansprüche, wobei man eine erschöpfte wässrige Salpetersäure aus dem Reaktor ablässt, sobald die Säurekonzentration unter einen vorgegebenen Grenzwert sinkt und/oder die Cobaltionenkonzentration über einen vorgegebenen Grenzwert steigt.

6. Verfahren nach einem der vorhergehenden Ansprüche, wobei man die Temperatur der wässrigen Salpetersäure von einer ersten Temperatur im Bereich von 10 bis 40 °C auf eine Endtemperatur im Bereich von 60 bis 80 °C erhöht.

7. Verfahren nach einem der Ansprüche 4 bis 6, wobei man einen außerhalb des Reaktors geführten Strom der wässrigen Salpetersäure erwärmt.

8. Verfahren nach einem der vorhergehenden Ansprüche, wobei man den Reaktor nach der Behandlung mit wässriger Salpetersäure mit einem Wasserstrahl reinigt, um die Entfernung der Cobaltablagerungen zu vervollständigen.

9. Verfahren nach einem der vorhergehenden Ansprüche, wobei man den Reaktor nach der Behandlung mit wässriger Salpetersäure mit Wasser oder Wasserdampf spült.

10. Verfahren nach einem der vorhergehenden Ansprüche, wobei man Cobalt aus der erschöpften wässrigen Salpetersäure zurückgewinnt, wobei man aus der erschöpften wässrigen Salpetersäure ein Cobaltsalz ausfällt, das Hydroxid- und/oder Oxidanionen enthält, das Cobaltsalz abtrennt und das Cobaltsalz mit Essigsäure zu einer Cobaltacetat-Lösung umsetzt.

## Claims

1. A method of removing cobalt deposits in a reactor for the cobalt-catalyzed high-pressure hydroformylation of olefins by treatment with aqueous nitric acid, wherein the reactor is at least partly filled with aqueous nitric acid, the acid concentration and/or the cobalt ion concentration of the aqueous nitric acid is/are measured during the treatment and, if the change in the acid concentration and/or the cobalt ion concentration over time drop(s) below a prescribed limit value, the temperature of the aqueous nitric acid is increased during the treatment.

2. The method according to claim 1, wherein a nitrogen oxide-comprising offgas formed during the dissolution of the cobalt deposits in the aqueous nitric acid is combined with a gas comprising molecular oxygen and the offgas is at least partly absorbed in an aqueous liquid.

3. The method according to claim 2, wherein the aqueous liquid is conveyed in countercurrent to the offgas in a scrubbing column and a gas comprising molecular oxygen is introduced into the offgas and/or into the scrubbing column.

4. The method according to any of the preceding claims, wherein the aqueous nitric acid is circulated in the reactor during the treatment.

5. The method according to any of the preceding claims, wherein an exhausted aqueous nitric acid is drained from the reactor as soon as the acid concentration drops below a prescribed limit value and/or the cobalt ion concentration rises above a prescribed limit value.

6. The method according to any of the preceding claims, wherein the temperature of the aqueous nitric acid is increased from a first temperature in the range from 10 to 40°C to a final temperature in the range from 60 to 80°C.

7. The method according to any of claims 4 to 6, wherein a stream of the aqueous nitric acid conveyed outside the reactor is heated.

8. The method according to any of the preceding claims, wherein the reactor is cleaned by means of a water jet after the treatment with aqueous nitric acid in order to complete the removal of the cobalt deposits.

9. The method according to any of the preceding claims, wherein the reactor is flushed with water or steam after the treatment with aqueous nitric acid.

10. The method according to any of the preceding claims, wherein cobalt is recovered from the exhausted aqueous nitric acid, wherein a cobalt salt comprising hydroxide and/or oxide anions is precipitated from the exhausted aqueous nitric acid, the cobalt salt is separated off and the cobalt salt is reacted with acetic acid to give a cobalt acetate solution.

## Revendications

1. Procédé d'élimination de dépôts de cobalt dans un réacteur pour l'hydroformylation haute pression catalysée par du cobalt d'oléfines par traitement avec de l'acide nitrique aqueux, dans lequel le réacteur est au moins partiellement rempli avec de l'acide nitrique aqueux, la concentration en acide et/ou la concentration en ions cobalt de l'acide nitrique aqueux sont mesurées au cours du traitement et, lorsque la modification temporelle de la concentration en acide et/ou de la concentration en ions cobalt diminue en dessous d'une valeur limite prédéterminée, la température de l'acide nitrique aqueux pendant le traitement est augmentée.

2. Procédé selon la revendication 1, dans lequel un gaz d'échappement contenant des oxydes d'azote, qui est formé lors de la dissolution des dépôts de cobalt dans l'acide nitrique aqueux, est réuni avec un gaz contenant de l'oxygène moléculaire et le gaz d'échappement est au moins partiellement absorbé dans un liquide aqueux.

3. Procédé selon la revendication 2, dans lequel le gaz d'échappement est mis en circulation dans une colonne de lavage à contre-courant du liquide aqueux et un gaz contenant de l'oxygène moléculaire est introduit dans le gaz d'échappement et/ou dans la colonne de lavage.

4. Procédé selon l'une quelconque des revendications précédentes, dans lequel l'acide nitrique aqueux est mis en circulation dans le réacteur pendant le traitement.

5. Procédé selon l'une quelconque des revendications précédentes, dans lequel un acide nitrique aqueux épuisé est déchargé du réacteur dès que la concentration en acide diminue en dessous d'une valeur limite prédéterminée et/ou dès que la concentration en ions cobalt augmente au-dessus d'une valeur limite prédéterminée.

6. Procédé selon l'une quelconque des revendications précédentes, dans lequel la température de l'acide nitrique aqueux est augmentée d'une première température dans la plage allant de 10 à 40 °C à une température finale dans la plage allant de 60 à 80 °C.

7. Procédé selon l'une quelconque des revendications 4 à 6, dans lequel un courant mis en circulation à l'extérieur du réacteur chauffe l'acide nitrique aqueux.

8. Procédé selon l'une quelconque des revendications précédentes, dans lequel le réacteur est nettoyé avec un jet d'eau après le traitement avec l'acide nitrique aqueux pour compléter l'élimination des dépôts de cobalt.

9. Procédé selon l'une quelconque des revendications précédentes, dans lequel le réacteur est rincé avec de l'eau ou de la vapeur d'eau après le traitement avec l'acide nitrique aqueux.

10. Procédé selon l'une quelconque des revendications précédentes, dans lequel le cobalt est récupéré à partir de l'acide nitrique aqueux épuisé, un sel de cobalt qui contient des anions hydroxyde et/ou oxyde étant précipité à partir de l'acide nitrique aqueux épuisé, le sel de cobalt étant séparé et le sel de cobalt étant mis en réaction avec de l'acide acétique pour former une solution d'acétate de cobalt.
